(19)

**Europäisches Patentamt**
**European Patent Office**
**Office européen des brevets**

(11)  **EP 1 250 886 B1**

(12)  **EUROPEAN PATENT SPECIFICATION**

(45) Date of publication and mention
of the grant of the patent:
**21.04.2010  Bulletin 2010/16**

(51) Int Cl.:
*A61B 5/0476* *(2006.01)*

(21) Application number: **01109804.3**

(22) Date of filing: **21.04.2001**

(54) **Anesthesia monitoring system based on electroencephalographic signals**

Anordnung zum Überwachen der Anästhesie auf Basis von elektroencephalographischen Signalen

Système de surveillance d'anesthésie à base de signaux électro-encéphalographiques

(84) Designated Contracting States:
**AT BE CH CY DE DK ES FI FR GB GR IE IT LI LU MC NL PT SE TR**

(43) Date of publication of application:
**23.10.2002  Bulletin 2002/43**

(73) Proprietor: **Hospira Sedation, Inc.**
**North Billerica, MA 01862 (US)**

(72) Inventors:
• **Ennen, David W.**
**Holliston, MA 01746 (US)**
• **Jiminez, Jorge R.**
**Acton, MA 01720 (US)**
• **Marro, Dominic P.**
**North Andover, MA 01845 (US)**

(74) Representative: **Frhr.Schenck zu Schweinsberg, Elard**
**Vossius & Partner**
**Siebertstrasse 3**
**81675 München (DE)**

(56) References cited:
**WO-A-00/02484      US-A- 5 377 100**
**US-A- 5 775 330      US-A- 5 813 993**

EP 1 250 886 B1

**Description**

FIELD OF THE INVENTION

The current invention relates to the field of medical anesthesia. More particularly it relates to the field of electronic monitoring of patients undergoing anesthesia, especially for use during and after surgical operations. The invention relates more specifically to the use of electroencephalograph (EEG) signals for electronically monitoring a patient's state of awareness.

BACKGROUND OF THE INVENTION

In current medical practice, at least for highly invasive surgery, a patient is placed under general anesthesia. Anesthesiology is a medical art practiced in the United States by and large by board certified physicians (anesthesiologists) and nurses (nurse anesthetists) specifically trained to administer anesthetic drugs and monitor patients under anesthesia. The state of patient anesthesia is attained by the controlled administration of various drugs with known anesthetic properties. These drugs cause the patient to lose consciousness, sensation, and motor control. The physician monitors the patient's state of awareness by means of a number of disparate clinical signs known empirically to provide useful and reliable information about the patient's state of unconsciousness.

Generally, the patient is anesthetized prior to surgery by the specialized medical practitioner (anesthesiologist or nurse anesthetist), who administers one or more vapors or gases by inhalation or introduces anesthetic drugs intravenously. Volatile substances include nitrous oxide, sevoflurane, desflurane, flurane and isoflurane, and halothane. Intravenous anesthetics include pentothal, evipal, procaine, nitrous narcotic with propofol induction, methohexital, and etomidate.

A correctly administered general anesthetic should remove any sensation of pain and any awareness of the operation itself. (Patients insufficiently deeply anesthetized have reported terror at becoming aware of the surgical procedure while paralyzed.)

The anesthetic should further disable the patient's motor control so that the patient cannot move. Otherwise, the patient may exhibit involuntary (reflex) muscle movements, which can disturb the area being surgically manipulated. Prevention of movement can be accomplished by anesthetic agents acting on the central nervous system or with a blockade of the neuromuscular junction with muscle relaxants.

Finally, the anesthesia must avoid depressing the patient's blood pressure so much as to reduce blood flow to the brain to a dangerous extent. Generally 50 mm Hg for mean arterial pressure is a lower limit.

A trained anesthesiologist or nurse anesthetist will monitor the patient's vital signs such as respiration and pulse rates, check the patient's pupil dilation, and check certain reflexes, such as the lash reflex, and other physiological signs to estimate the depth of anesthesia. In some instances, however, either the practitioner does not have access to all of the required clinical information or other circumstances intervene. For example, in some procedures the patient is draped in such a way as to make observation of some clinical indicators difficult or impossible. In addition, in very lengthy procedures the attention of even the best practitioner can flag.

In such circumstances it would frequently be useful to have an electronic monitor to track the patient's level of consciousness. In particular, it sometimes would be useful to have an instrument, which, once the plane of anesthesia is established qualitatively by the anesthesiologist using traditional clinical indicators, would indicate significant changes in the patient's state of anesthesia or patient responses to stimuli, which would indicate insufficient anesthesia.

A number of inventors have developed systems for using EEG signals, generally in combination with other signals, to monitor anesthesia, sleep, or other states on the consciousness-unconsciousness continuum. Kaplan et al., U. S. Patent No. 5,813,993, issued September 29, 1998, disclosed a drowsiness detection system based on EEG signals. This invention relies heavily on frequencies in EEG signals above 30 Hz. It does not use any form of norming and in addition applies an ad hoc weighted sum of inverted spectral power coefficients. Maynard, U. S. Patent No. 5,816,247, issued October 6, 1998, uses a combination of time domain amplitude envelope analysis and frequency analysis in conjunction with a trainable neural network to classify awareness and sleep states. Kangas et al., U. S. Patent No. 5,775,330, issued July 7, 1998, uses transform processing and neural net analysis to classify states of anesthesia. The output of the neural net could be used to produce a single index of awareness. However, all of these prior art systems either represent an unnecessary level of complexity or an absence of empirical basis or both.

A prior patent to John, U.S. Patent No. 5,699,808, issued December 23, 1997, discloses a system to monitor multiple patients simultaneously in the surgical recovery room or in intensive care. This system, however, combines certain features of EEG signals and other features including those of evoked potentials to arrive at an estimate of the patient's state of consciousness. It specifically incorporates the use of electrocardiograph (EKG) and electromyograph (EMG) electrodes and also input from a blood pressure detector and from a respiration monitor. This prior art system also requires evoked potentials, specifically Brainstem Auditory Evoked Response (BAER) and Brainstem Somatosensory Evoked Response (BSER). Use of evoked potentials, however, involves the use of additional disposables and a longer set-up time. Further, this system relies very heavily on self-norming and in particular on updating self-norming depending on the state of the patient.

An earlier patent to the same inventor, John, U.S. Patent No. 4,557,270, issued December 10, 1985, suffered from additional and more severe limitations since it required measurement of blood temperatures and volumes. Finally, John, U.S. Patent No. 4,545,388, issued October 8, 1985, disclosed the basic process of self-norming of processed EEG data.

Another inventor, Prichep, U.S. Patent No. 5,083,571, issued January 28, 1992, disclosed a significant advance in the utilization of EEG signals for diagnostic purposes. Prichep disclosed the use of discriminant analysis to sharpen the diagnostic capability of quantities derived from EEG signals with respect to certain well-known diagnostic categories of psychiatric illness. This work compared quantities derived from a patient with parameters derived from populations of persons thought to suffer from specific identified illnesses.

US-A-5 377 100 discloses a system utilizing a plurality of modules comprising observers configured to produce measures of specific characterisics in EEG signals, as well as an observer mediator that produces an output parameter charaterizing the patient's state of awareness.

Finally, a prior application which has not yet issued, John, U.S. Patent No. 6,067,467 issued May 23, 2000, applied discriminant analysis to the statistical differentiation of unconscious from conscious states from EEG signals. However, this invention relied heavily on BAER and BSER signals and self-norming. In addition, this invention stated, with respect to Chamoun, No. 5,010,891, issued April 30, 1991, that "the comparison of patients with a normal group, in itself, is not believed to provide reliable information in the surgical context of determining if a patient will be sufficiently anesthetized." App. at p. 4. Since that time, however, the current inventors have learned from further investigation and experimentation that population-norming is sufficiently reliable and self-norming adds unnecessarily to the complexity of the system without adding to performance. What is therefore most lacking in all of these prior art inventions is simplicity and cost effectiveness.

It is therefore an object of the current invention to provide an EEG based anesthesia monitoring system that completely avoids use of transducers for and inputs from other than EEG signals, that is, avoids the use of pulse, blood pressure, and respiration rate sensors and leads. It is a further object of this invention to provide an anesthesia monitoring system based on EEG signals, which completely avoids the need for BAER and BSER stimulation and response monitoring. It is a further object of this invention to provide an EEG based anesthesia monitoring system which dispenses with the cumbersome and error prone process of self-norming. It is another object of this invention to provide an EEG based anesthesia monitoring system which utilizes sample population-norming.

## SUMMARY OF THE INVENTION

The current invention comprises a system for using EEG signals to monitor the state of anesthesia of a patient at various stages preparatory to, during, and after administration of anesthetic and surgical operation, and in intensive care during recovery from the operation and anesthesia. The system comprises a headset attached to a patient, a patient module connected to the headset, apparatus for transmitting EEG signals to an analysis unit, and the analysis unit itself, according to claim 1. The analysis unit further comprises a number of subsystems, but its essence is the Algorithm which processes the EEG signals into a parameter usable to estimate and/or track the patient's state of unconsciousness or consciousness while under anesthesia.

The primary function of the analysis unit is to classify anesthetized patients according to their conscious state, as determined from an analysis of volunteer data using the OAA/S scale. The version of this scale used in this invention is:

| Modified Observer's Assessment of Alertness/Sedation Scale | |
|---|---|
| **Response** | **Score** |
| Responds readily to name spoken in a normal tone | 5 |
| Lethargic response to name spoken in a normal tone | 4 |
| Responds only after name is called loudly and/or repeatedly | 3 |
| Responds only after mild shaking or prodding | 2 |
| Responds only to noxious stimulus and not to mild shaking or prodding | 1 |
| Does not respond to noxious stimulus | 0 |
| Burst Suppression | -1 |

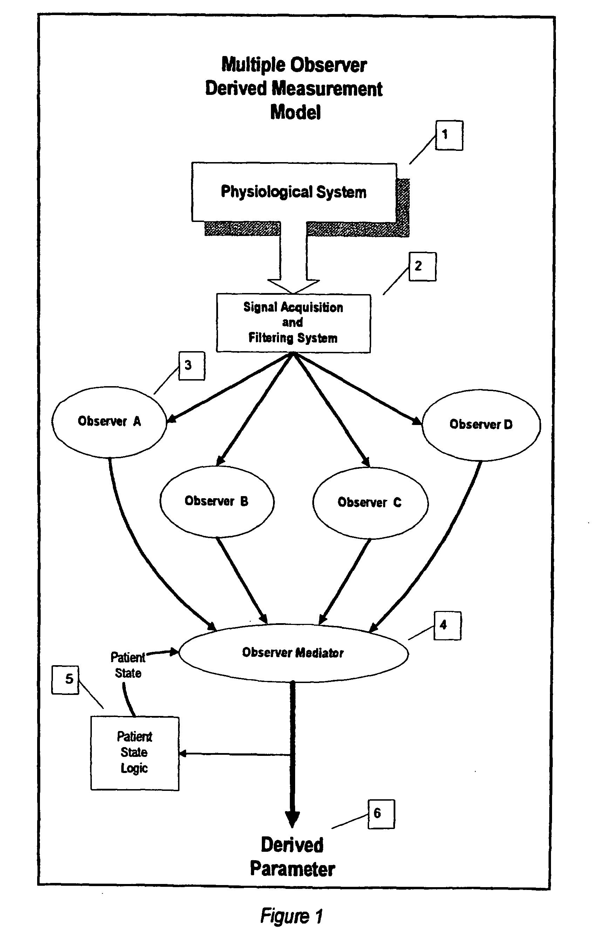

The design of the analysis unit is based on the Multiple Observer Derived Measurement Model depicted in Figure 1. An observer is a thread of execution and logic, an algorithm, which processes a stream of data and generates a measure of a characteristic(s) identified within the data stream. The principle is that is easier to construct individual observers tuned to specific characteristics in the data stream, than to create one observer that is tuned to classify an ensemble of characteristics in the data stream. Observers 3 are classifier functions that detect signatures within the information. These signatures may be in the time domain, frequency domain, or a combination of the two domains. By tuning observers to specific signatures, selective filtering can be employed to improve the accuracy and latency of an observer. What may be noise to one observer may be critical information to another. This selective filtering increases the overall utilization of the acquired physiological information and thereby improving the performance of the final derived measure.

An Observer Mediator 4 is responsible for logically combining these individual observations in to the single Derived Parameter. The Observer Mediator can weigh the individual observations by monitoring each observer's input signal quality and the context of the observation based on the patient state. The patient state is derived from the behavior of the derived parameter over time and this is fed back to the Observer Mediator. Functionally, either on demand or on a periodic basis, the Observer Mediator polls the Observers and based on patient state and the 'quality' of the individual observations, combines the observations into a single derived measure. The Derived Parameter may be enhanced in sensitivity or scope by either further tuning of established Observers or adding additional Observers.

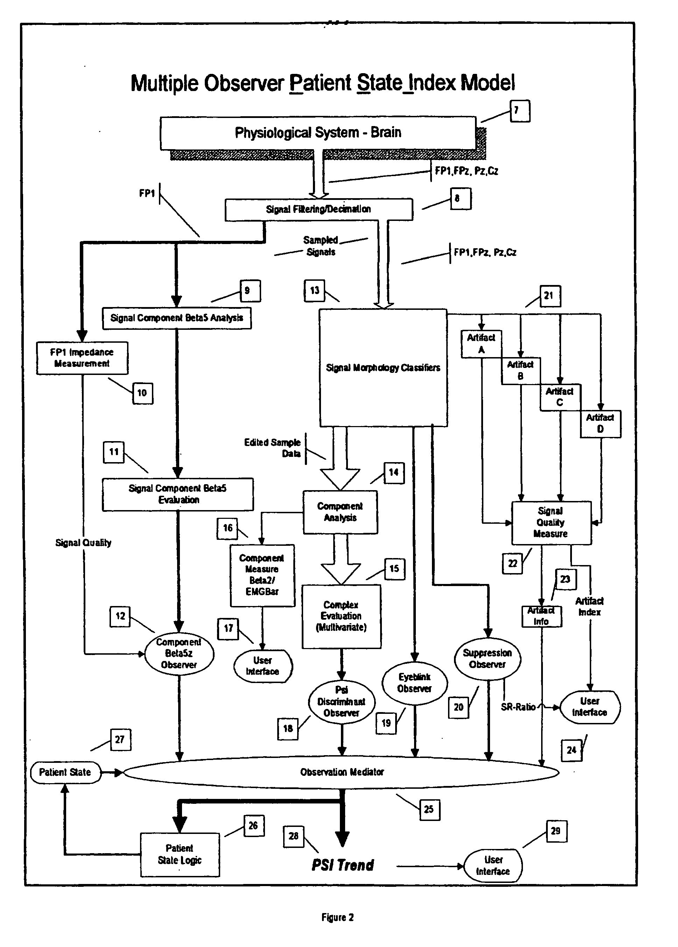

The primary output of the PSA 4000 algorithm is a single derived parameter called the Patient State Index (PSI) that maps to the OAA/S scale independent of anesthetic agent. The implementation of the Multiple Observer Model for this measure of state of consciousness, the PSI, is shown in Figure 2. In this system, electroencephalograph (EEG) signals are acquired from an array of electrodes on the patient's scalp. These raw EEG signals are filtered and decimated to reduce external noise and to satisfy data sampling rate (Nyquist) requirements.

Following decimation, artifact analysis takes place. The EEG data is analyzed for validity and contamination. This step results in the setting of various artifact codes. After artifact analysis, the Eye-blink Observer and the Suppression Observer operate. This is followed by the calculation of the Artifact Index and the Suppression Ratio Index. For the FFT calculations, The Fast Fourier Transform (FFT) of the EEG data is calculated for each of the four channels. At this point, the system decomposes the Frequency Spectrum. In this operation the FFT spectral data is divided into frequency bands. After this, the EMG Index is calculated, and the EMG Beta-5 observer operates. The Discriminant Observer then makes its calculations, resulting in a Probability of Correct Classification based on parameters derived from sample populations. Finally, the Observer mediator combines the probability with other the output of the other three observers to the Patient State Index (PSI).

Further processing analyzes information in the 0.5 Hz to 50 Hz frequency range. As shown in Figure 2, the sample data streams are divided into two primary streams: the FP1 channel is separately processed by the Beta5 Observer, all channels [FP1, FPz', Pz, Cz] are processed by an ensemble of signal morphological classifiers 13 (artifact detectors). By continuously monitoring the impedance of the FP1 electrode 10 the Beta5 Observer's signal quality can be assessed. The FP1's signal quality is combined with Beta5 analysis 9 and evaluation 11 by the Beta5 Observer and propagated to the Observation Mediator. The outputs of the Signal Morphology Classifiers are four artifact free EEG data streams and a declaration of the types of artifacts detected. Two of the artifact classifiers propagate information to Observers. The Eyeblink Observer 19 is notified of the number and types of eyeblinks detected in the four EEG channels. The Suppression Observer 20 is notified whether EEG suppression has been detected over the last time period. The artifact free EEG data is further processed by the PSI Discriminant Observer 18, which performs a more complex multiple component analysis that serves as the foundation of the consciousness algorithm. The four observations: Beta5, PSI Discriminant, Eyeblink and Suppression) are propagated to the Observation Mediator 25. The Mediator combines these observations with measures of signal quality and appropriateness of observations based on patient state into an update of the Patient State Index and the associated trend. The time course of the PSI is monitored and logic is applied to assess the patient's state and this information is fed back to the Observation Mediator. It is through the use of this Multiple Observer Model that a clinically functional measure of state of consciousness is realized.

The output of the Algorithm is a set of four processed parameters calculated every 2.5 seconds (each 2.5 second block is referred to as an epoch). These are the main output parameter, i.e., the Patient State Index (PSI); the Suppression Ratio (SR); the EMG index (EMG); and the Artifact Index (ART). The measures in addition to the PSI provide additional information to the instrument operator on either specific aspects of the patient state or data quality. These measures are shown in Figure 2 to be directed to the User Interface. The Artifact Index is a measure of signal quality. The SR-Ratio is the percentage of time in the last minute the patient's EEG has been suppressed. The Beta2 component measure is related to the degree of muscle activity (EMG) detected. The outputs of this multiple observer based PSA 4000 algorithm is a periodic update of: the Patient State Index (the primary derived parameter), the Artifact Index, the Suppression Ratio and a measure of EMG activity.

The Patient State Index 164, the primary indicator of patient level of awareness, is developed to characterize the relative state of consciousness of an anesthetized patient. The Algorithm outputs a periodic update of this primary parameter. The Algorithm provides for upper and lower thresholds of this parameter within which the patient will be said to be in an appropriate level of unconsciousness for surgery. (Other levels may be appropriate for other conditions such as intensive care sedation.) The PSI range is defined to be from 0 to 100, with higher values indicating a higher level of consciousness or awareness.

The Suppression Ratio **162** is an indicator of the relative amount of time that the patient's EEG waveforms exhibit a characteristic Burst Suppression pattern. The Burst Suppression pattern is accepted to be an indicator of deep levels of unconsciousness under sedation. In certain situations of traumatic head injury, for example, it is necessary to reduce the brain's need for oxygen by putting the patient into a drug induced (barbiturate) coma. This brain state is observed in the EEG as Burst Suppression. For most surgical procedures, burst suppression is considered an inappropriately deep level of sedation where the anesthesiologist would normally reduce drug flow rates accordingly. The Suppression Ratio is the percentage of epochs (2.5-second epochs) in the last one minute that have been declared as Suppressed Epochs.

The EMG Index **163** is an indicator of muscle activity. Under certain conditions, an EMG response may be interpreted as an indicator of the patient's response to pain or stress. The anesthesiologist's action would depend upon the conditions present when the EMG response occurs, as EMG is a normal indication at the end of surgery. During surgery, the anesthesiologist titrates additional hypnotic for stress or analgesic for pain accordingly. The EMG Index is a weighted percentage of half-epochs (over the past one minute) in which muscle activity, as measured by the power in the BETA-2 band, exceeds a threshold level. Newer epochs are weighted more heavily than the older ones.

The Artifact Index **165** is an indicator of data quality, or of the amount of artifacts present in the data. It is also a weighted percentage (over the past one minute). Increase in the artifact index is normal during any patient movement and may be associated with the use of certain equipment such as BOVI or train-of-four when applied to the face. Poor contact impedance aggravates all sources of artifact and will require intervention by the anesthesiologist to correct poor electrode contact with the patient.

## BRIEF DESCRIPTION OF THE DRAWINGS

Figure 1 portrays the basic structure of the Multiple Observer Model.

Figure 2 is a more detailed illustration of the Multiple Observer Model.

Figure 3 shows the basic structure of the system

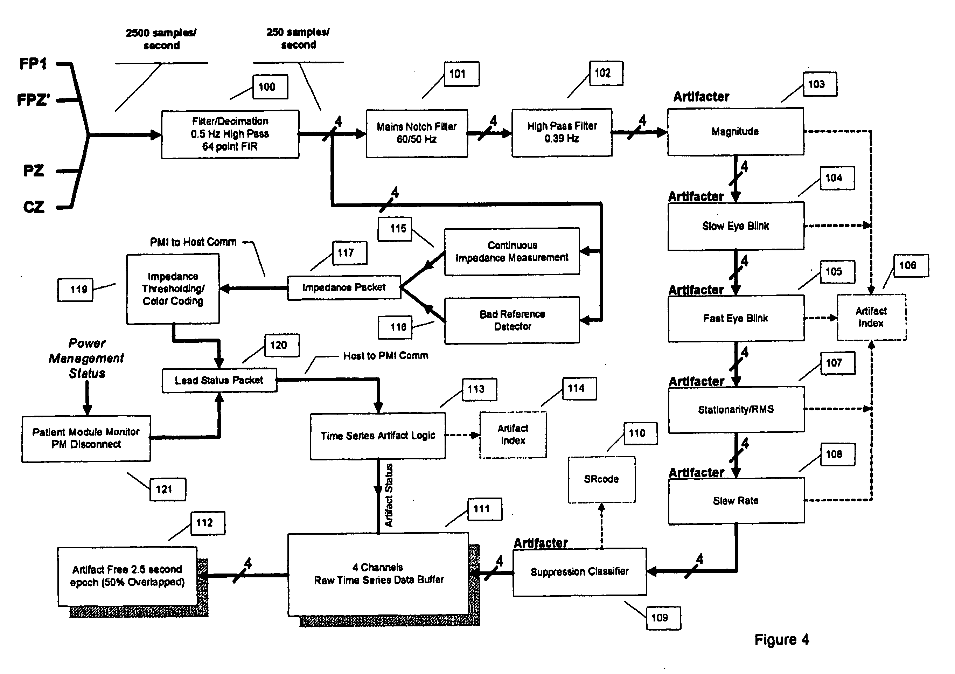

Figure 4 shows the basic logical flow of the Algorithm.

Figure 5 continues and supplements the logical flow diagram of the Algorithm.

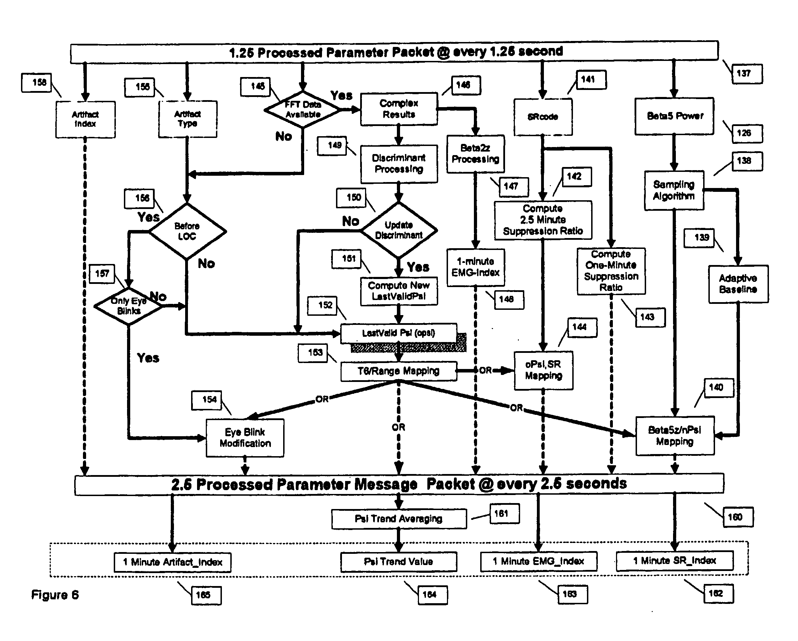

Figure 6 shows the final stages of the logical flow of the Algorithm.

Figure 7 illustrates data flow between buffers

## DETAILED DESCRIPTION OF THE PREFERRED EMBODIMENT

THE HEADSET AND THE PATIENT MODULE

In a related previous application, No. 09/113,946, filed July 10, 1998, incorporated herein by reference as though fully set forth, one of the current inventors and others described a head set which can extract from the patient's head EEG signals from five favored locations of the set of international standard locations. The five favored locations are denoted in the international system by Fp1, Fp2, Fpz', Cz, and Pz. In current embodiments these are electrically referenced to linked ear or linked mastoid contacts. EEG data from four of these five specific locations are analyzed. Alternatively, a more elaborate headset, such as that disclosed by Imran, U.S. Patent No. 5,479,934, issued January 2, 1996, can be used to obtain information from the four desired locations.

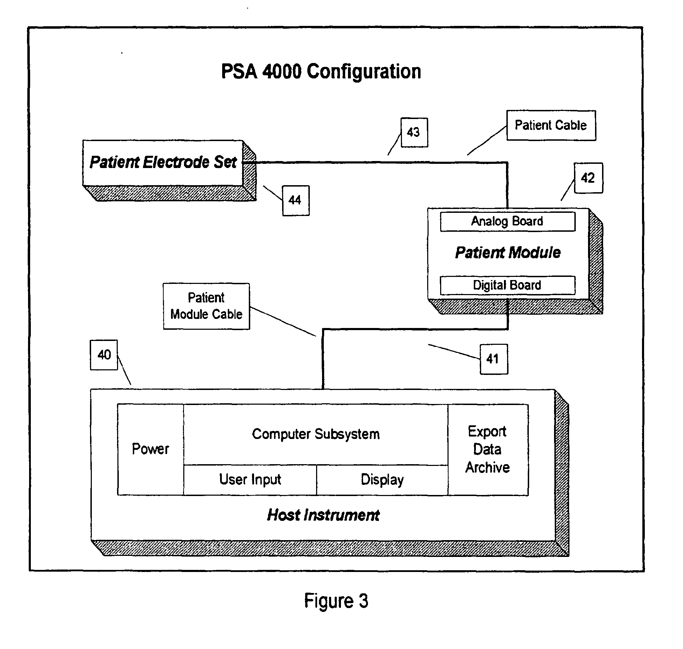

As shown in Figure 3, the PSA Patient Interface consists of a Patient Module **42**, patient interface cable **43**, and a Patient Electrode Set **44** designed to provide a superior quality, programmable patient interface for EEG monitoring in the OR and ICU. The Patient Module is housed in a custom molded plastic enclosure with an integral universal-mounting bracket that facilitates attachment to an IV pole, bed sheet or rail. A detachable patient interface cable provides a quick connect / disconnect capability to the PSA appliance or Patient Module. EEG signals from the appliance are acquired with an isolated instrumentation grade, 4-channel pre-amplifier assembly and programmable multiplexed high speed A/D converter. The signal inputs are acquired referentially with reformatting provided by the Host application if necessary. Preamplifier optimization for EEG is standard, with EP and ECG optional by design. The combination of optically isolated data pathways, a low leakage / high isolation power converter and amplifiers with precise gain and band-pass matching results in greater than 120 dB CMRR. Calibration, Impedance Test, and Normal Operation are remotely controlled through the DSP Interface using commands generated by the Host Application. A full duplex connection is provided between the Patient Module and the DSP via dual optical-isolators that comply with VDE0884 for safety with extremely low leakage. The power converter is a UL Listed & Medical Grade. This extreme isolation results in negligible leakage currents and assures IEC601 / UL2601 compliance with superior common mode performance.

The proprietary ISA bus DSP card provides a real time interactive link between the host and patient module and manages the acquisition, calibration and impedance functions of the patient module. Balanced differential drivers are used to minimize EMI associated with serial data transmission while providing the ability to extend the link to approximately 1000 feet. Filtering and decimation of the acquired data takes place in the DSP.

**THE ANALYSIS UNIT**

The PSA 4000 analysis unit embodies and operates by means of a complex Algorithm referred to hereafter as the PSA 4000 Algorithm. The system operates in three distinct modes, sometimes referred to as states, with different characteristics. The three states are labeled as follows: 1) Data Accumulation; 2) Awake Patient; and 3) Unconscious Patient. Two very specific and well-defined events cause the transition of the system among these four states. These events are labeled as: 1) Sufficient Data Accumulated ; and 2) Loss of Consciousness . The identification of events and the switching among the states at the occurrence of an identified event is described further below following the description of the operation of the PSA 4000 Algorithm.

The following notation is used in describing EEG data:

| Sample | A sample is actually a set of four values, one for each of the electrode, associated with a particular instant of time. |
| --- | --- |
| j | All samples have a sample index, denoted by j that increases with time. |
| j=0 | The index of the most recent sample has index 0. All other samples therefore have a negative index. |
| $t_j$ | The time associated with a particular sample set is denoted by $t_j$. The time resolution of the algorithm neglects the miniscule differences between time values of electrode values in a sample. |
| $S_j$, $S(j)$ | The sample associated with a particular index j. |
| $S(t_j)$ | The sample associated with a particular time. |

The basic operational modules of the PSA 4000 Algorithm are: EEG Data Collection, Filtering and Decimation; Artifact Detection and Signal Morphology Analysis; Eye-blink Observation; Suppression Observation; Calculation of Artifact Index; Calculation of Suppression Ratio Index; FFT Calculation; Spectral Band Decomposition; Calculation of the EMG Index; EMG Beta-5 Observation; Discriminant Observer Calculation of the Probability of Correct Classification; Observer Mediation for the PSI; and Display of the PSI, the Suppression Ratio Index, the EMG Index, and the Artifact Index.

***Operational Modules***

1) EEG Data Collection, Filtering and Decimation

The patient module (PM) **42** acquires EEG data at a sampling rate of 2500 Hz. The sampling and processing are established to produce a frequency representation resolution of 0.25 Hz or better.

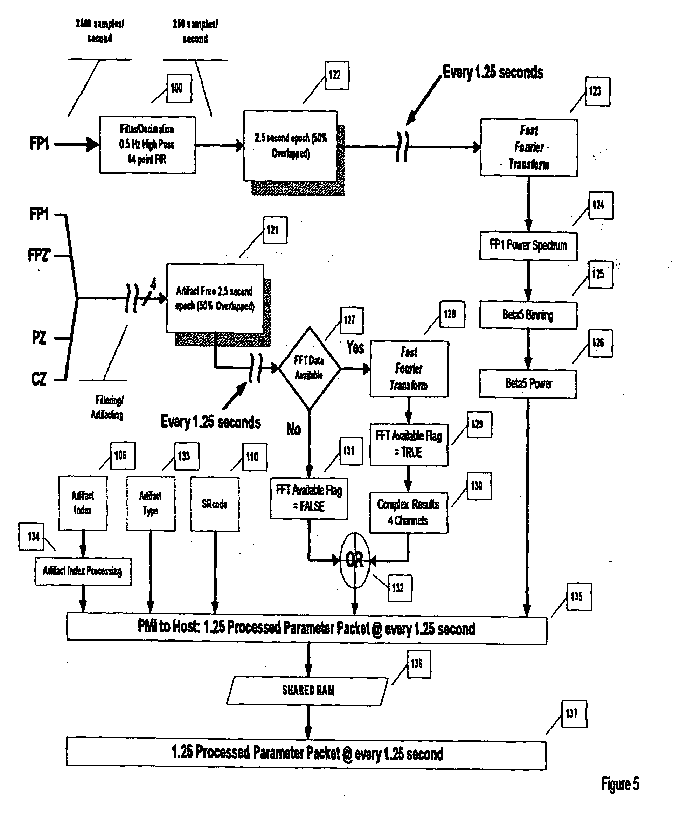

Data on four channels from the headpiece are filtered and decimated by a 10-to-1 low pass decimation filter **100,** as shown in Figure 5. This is done every 10 samples resulting in 1 sample every 1/250 sec., for an effective sampling rate of $f_s$ = 250 Hz. After the 10-to-1 decimation and filter, the EEG data passes through a high-pass filter **102** with a cut-off frequency of $f_h$ = 0.4 Hz. The most recent $L_1$ samples are used, where $L_1 = f_s/f_h$ = 625. Filtering starts at the sample s(-1/2L1) i.e., samples more recent than s(-1/2L1) are not filtered. The average of the most recent L1' - 625 samples is subtracted from sample s(-

$$s'(-\tfrac{1}{2}L_1) = s(-\tfrac{1}{2}L_1) - \frac{1}{L_1}\sum_{j=-L_1+1}^{0} s'(j) \qquad\qquad \mathbf{1}$$

1/2L1). We denote the filtered sample by s'(-1/2L1):

The filtered sample is stored in the buffer as shown in Figures 8 and 9 and refreshed every half epoch.

2) Artifact Detection and Signal Morphology Analysis

Artifact detection and analysis are performed once every sample, i.e., 250 times per second in an Artifacter Bank **103-108**. This analysis results in an artifact type being associated with every sample that is classified as being affected by an artifact. Artifact types are also collated on an epoch-by-epoch basis. Artifact analysis is performed only *on filtered* samples. As shown in Figure 5, after the high-pass filter, the artifact engine analyzes data on four channels for 5 kinds of artifacts:

    a. Magnitude **103**
    b. Slow Eye Blink **104**
    c. Fast Eye Blink **105**
    d. Slew rate **107**
    e. Stationarity **108**.

These are later combined in an Artifact Index module **106**.

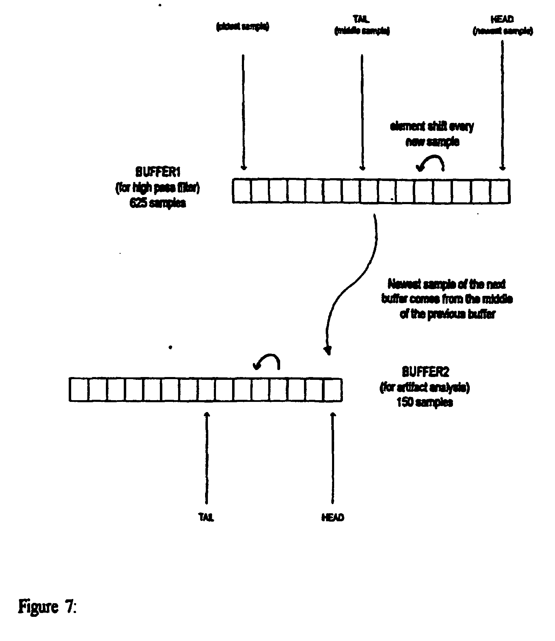

As is illustrated in **Figure 7**, artifact analysis is done on a series of sample sets (sample buffers) of varying sizes. The magnitude, suppression, slew rate, and eye blink artifacts are checked on a buffer of 150 samples. These 150 samples (0.6 seconds) are older than the latest 312 of the 625 samples that have gone through the high-pass filter. Thus, they are identical with the newest 150 samples in the *older half* of the high-pass filter buffer. Similarly, the rms deviation artifact is checked on the 2000 samples (8.0 seconds) before (older than) the latest 75 of the 150 samples that were checked for the previous artifact types.

The conditions used in checking for the various types of artifacts are:

**i) Magnitude**

The magnitude of the newest *filtered* sample is checked, on each of the four channels **103.** (Recall that the *latest filtered* sample is older than the latest sample by L1/2 samples.) If the magnitude of at least one of the channels exceeds a set threshold, the sample

$$\left| s'(-\tfrac{1}{2}L_1) \right| > M_{thresh} \qquad\qquad \mathbf{2}$$

is classified as a magnitude artifact. Thus the condition is
where $M_{thresh}$ is the magnitude threshold. The magnitude threshold is different for different channels and is determined empirically.

**ii) Slew Rate**

The slew rate detector **107** checks for sudden changes in the magnitude of samples. The rate of change cannot be greater than 15 $\mu$V over 20 ms. To check this, the detector obtains the largest sample and the smallest sample over the $\delta_{slew}$ samples *older* than s' ($-\tfrac{1}{2}L_1-\tfrac{1}{2}L_2$). If the difference between sample s' ($-\tfrac{1}{2}L_1-\tfrac{1}{2}L_2$) and either the largest or the smallest sample is greater than 15 $\mu$V, than a slew rate artifact is declared. Mathematically, the conditions can be expressed as:

$$s'(-\tfrac{1}{2}L_1 - \tfrac{1}{2}L_2) - \min(-\tfrac{1}{2}L_1 - \tfrac{1}{2}L_2 - \delta_{slew}, -\tfrac{1}{2}L_1 - \tfrac{1}{2}L_2) > L_{thresh} \qquad\qquad \mathbf{3}$$

$$\max(-\tfrac{1}{2}L_1 - \tfrac{1}{2}L_2 - \delta_{slew}, -\tfrac{1}{2}L_1 - \tfrac{1}{2}L_2) - s'(-\tfrac{1}{2}L_1 - \tfrac{1}{2}L_2) > L_{thresh} \qquad \textbf{4}$$

where $L_{thresh}$ is threshold for the slew rate artifact, equal to 15 $\mu$V. If either one of these conditions is satisfied, a slew rate artifact is declared.

### iii) Stationarity

The stationarity artifact is checked **108** on the $L_3$ = 2000 samples (8.0 seconds) before (older than) sample s' $(-\tfrac{1}{2}L_1 - \tfrac{1}{2}L_2)$. First the sum of the *squares of the samples* (not the squares of the deviations) is calculated. This is compared to the sample in the middle of the 2000 samples s' $(-\tfrac{1}{2}L_1 - \tfrac{1}{2}L_2 - \tfrac{1}{2}L_3)$. The following condition is checked:

$$s'(-\tfrac{1}{2}L_1 - \tfrac{1}{2}L_2 - \tfrac{1}{2}L_3) > \text{bigger}\left( f_{scale} \sum_j [s'(j)]^2, f_{limit} \right) \qquad \textbf{5}$$

If this condition is satisfied for a given scale factor and limit term then a stationarity artifact is declared.

3) Eyeblink Observation

The conditions for eye blinks, **104** and **105**, are checked only if the slew rate artifact is not detected because the slope required in a slew rate artifact is larger than the slope required for eye blinks The eye blink observer is mathematically similar to the slew rate detector, however, it checks for both positive and negative slopes together, i.e., it checks for EEG humps within certain parameters.

First, the observer checks for the conditions on the rise (the first half of the eyeblinks). For small eye blinks, the artifactor checks the $\delta_{EBSb}$ samples *older* than sample s' $(-\tfrac{1}{2}L_1 - \tfrac{1}{2}L_2)$ and obtains the largest and smallest samples. The following conditions are

$$s'(-\tfrac{1}{2}L_1 - \tfrac{1}{2}L_2) - \min(-\tfrac{1}{2}L_1 - \tfrac{1}{2}L_2 - \delta_{EBSb}, -\tfrac{1}{2}L_1 - \tfrac{1}{2}L_2) > L_{thresh} \qquad \textbf{6}$$

checked:
**or**

$$\max(-\tfrac{1}{2}L_1 - \tfrac{1}{2}L_2 - \delta_{EBSb}, -\tfrac{1}{2}L_1 - \tfrac{1}{2}L_2) - s'(-\tfrac{1}{2}L_1 - \tfrac{1}{2}L_2) > L_{thresh} \qquad \textbf{7}$$

If at least one of these conditions is satisfied, then the conditions on the second half of the eye blink are checked. The observer checks the $\delta_{EBSa}$ *filtered* samples *newer* than sample s' $(-\tfrac{1}{2}L_1 - \tfrac{1}{2}L_2)$ and obtains the largest and smallest samples. One of the

$$s'(-\tfrac{1}{2}L_1 - \tfrac{1}{2}L_2) - \min(-\tfrac{1}{2}L_1 - \tfrac{1}{2}L_2, -\tfrac{1}{2}L_1 - \tfrac{1}{2}L_2 + \delta_{EBSa}) > L_{thresh} \qquad \textbf{8}$$

**or**

$$\max(-\tfrac{1}{2}L_1 - \tfrac{1}{2}L_2 - \delta_{EBSb}, -\tfrac{1}{2}L_1 - \tfrac{1}{2}L_2) - s'(-\tfrac{1}{2}L_1 - \tfrac{1}{2}L_2) > L_{thresh}. \qquad \textbf{9}$$

following conditions must be satisfied:

If (Equation 8 or Equation 9) AND (Equation 10 or Equation 11) is satisfied,

$$s'(-\tfrac{1}{2}L_1 - \tfrac{1}{2}L_2) - \min(-\tfrac{1}{2}L_1 - \tfrac{1}{2}L_2 - \delta_{EBLb}, -\tfrac{1}{2}L_1 - \tfrac{1}{2}L_2) > L_{thresh} \qquad \mathbf{10}$$

then a small eye blink is declared. Similarly, for large eye blinks, the conditions are:

$$\max(-\tfrac{1}{2}L_1 - \tfrac{1}{2}L_2 - \delta_{EBLb}, -\tfrac{1}{2}L_1 - \tfrac{1}{2}L_2) - s'(-\tfrac{1}{2}L_1 - \tfrac{1}{2}L_2) > L_{thresh} \qquad \mathbf{11}$$

or

$$s'(-\tfrac{1}{2}L_1 - \tfrac{1}{2}L_2) - \min(-\tfrac{1}{2}L_1 - \tfrac{1}{2}L_2, -\tfrac{1}{2}L_1 - \tfrac{1}{2}L_2 + \delta_{EBLa}) > L_{thresh} \qquad \mathbf{12}$$

AND

$$\max(-\tfrac{1}{2}L_1 - \tfrac{1}{2}L_2, -\tfrac{1}{2}L_1 - \tfrac{1}{2}L_2 + \delta_{EBLa}) - s'(-\tfrac{1}{2}L_1 - \tfrac{1}{2}L_2) > L_{thresh} \qquad \mathbf{13}$$

or

If (Equation 12 or Equation 13) AND (Equation 14 or Equation 15) is satisfied, then a large eyeblink is declared. The threshold parameters for eye blinks are determined empirically.

4) Suppression Observation

Every time a *new filtered* sample is obtained, the suppression detector **109** looks at the sum of the squared deviations of samples over the latest *filtered* 600 milliseconds (150 samples, denoted by $L_2$) and over the *latest filtered* 20 milliseconds (5 samples, denoted by $L_5$).

$$\Delta(j_1, j_2) = \left( \sum_{j=j_1}^{j_2} [s'(j)]^2 \right) - \frac{1}{N(j_1, j_2)} \left[ \sum_{j=j_1}^{j_2} s'(j) \right]^2 \qquad \mathbf{14}$$

For reference, the sum of the squared deviations, generically defined, is: where $j_1$ and $j_2$ are sample indices, and $N(j_1, j_2)$ is the number of samples between these indices. This quantity is related to variance of the same sample set (the *average* of the sum

$$\sigma^2_{j_1, j_2} = \frac{\Delta(j_1, j_2)}{N(j_1, j_2)} \qquad \mathbf{15}$$

of the squared deviations) through

The difference between these two sums of squared deviations is intended as an approximate measure of the power in the band between 1.67 Hz and 50 Hz. If, on any one of the four channels, this power is less than a set threshold, then a suppression-type

$$\Delta(-\tfrac{1}{2}L_1 - L_2, -\tfrac{1}{2}L_1) - \Delta(-\tfrac{1}{2}L_1 - L_5, -\tfrac{1}{2}L_1) < S_{thresh} \qquad\qquad \mathbf{16}$$

artifact is associated with that sample. Thus the suppression condition is
where $S_{thresh}$ is the threshold for the Suppression Artifact. The thresholds are different for the different channels and are determined empirically.

**(a) Persistent Suppression Ratio (SR)**

The suppression Observer calculates a quantity called the persistent Suppression Ratio (pSR). It is defined as the percentage, over the past 2.5 minutes, of 2.5-second epochs in which a suppression artifact was detected. The pSR is used later in calculating the PSI from the PCC.

The pSR is also calculated based on 2.5-sec suppressed epoch declarations, even though 1.25-sec suppressed epoch declarations are available from the current overlapping-FFT scheme. (Recall that the connecting rule is, if one of the two 1.25-sec epochs in a 2.5-sec epoch is declared suppressed, then the 2.5-sec epoch is declared suppressed.) The pSR is used later in calculating the PSI from the PCC.

5) Calculation of Artifact Index

The 2.5-second Artifact Index **106** is one of the four final output parameters communicated to the user of the PSA 4000. The Artifact Index is a time-weighted percentage of 48 overlapped epochs (1.25-second epochs) in the past one minute that were declared as artifacted epochs. The newer half-epochs are weighted more heavily than the older half-epochs. The Artifact Index is calculated every 1.25 seconds.

6) Calculation of Suppression Ratio Index

The Suppression Ratio (SR) **143** is also one of the four final output parameters communicated to the user of the PSA 4000. The SR is defined as the percentage, over the past one minute, of 2.5-second epochs in which a suppression artifact was detected.

7) FFT Calculation

Whenever 625 continuous good (non-artifacted) samples are calculated, the FFT of the time series (i.e., the set of 625 samples, also called an epoch) is calculated. A Hamming Window is applied to the time series data before the calculation of every FFT (see references). EEG data is sliced into 2.5-second periods, called epochs, containing 625 samples each. The Fourier Transform of an epoch of EEG data is calculated. FFT calculations **126, 128** are done using a 50% overlap scheme, i.e., FFT calculations are done every 1.25-seconds for data covering the last good 2.5-second period.

8) Spectral Band Decomposition

The following band definitions are used in succeeding Algorithmic calculations **146**. The band definitions are given in units of Hertz (Hz).

Table 1

| Band Name | Band definition (Hz) |
|-----------|----------------------|
| $\Delta$ | 1.5 - 3.5 |
| $\theta$ | 3.5 - 7.5 |
| $\alpha$ | 7.5 - 12.5 |
| $\beta$ | 12.5 - 25.0 |
| $\beta_2$ | 25.0 - 50.0 |
| $\beta_5$ | 35.0 - 50.0 |
| tot | 1.5 - 25.0 |

9) Calculation of the EMG Index

The EMG Index **148** is an indicator is a time-weighted percentage of 1.25-second epochs in the past one minute that had an $F_1\beta_2$ z-component of greater than 1.96. The newer half-epochs are weighted more heavily than the older half-epochs.

10) EMG BETA-5 observation

The $F_1\beta_5$ raw measure **126,** is the power on the $F_1$ channel in the $\beta_5$ band. The $F_1\beta_5$ Z-component is the logarithm of the raw measure, and the $F_1\beta_5$ Z score is the population-normed Z-component. The $F_1\beta_5$ index is defined as a running average of the $F_1\beta_5$ Z-scores over the past twelve overlapped-epochs, in which the newest Z-score is limited to a maximum change of six population standard deviations from the latest running average.

11) Discriminant observer calculations

A discriminant **149** is a function of statistical variable that maximizes the separation, in the variable space, of two groups of interest. It is usually a linear combination of the statistical variables. Thus, specification of the discriminant involves both specification of the variables and their weights.

**i) Raw Measures for the Discriminant Observers**

The Raw Measures used by the discriminant observer **18** are defined by the following table:

Table 1:Raw Measures as a combination of electrodes and bands

|  | tot | $\Delta$ | $\theta$ | $\alpha$ | $\beta$ | $\beta2$ |
|---|---|---|---|---|---|---|
| $FP_1$ | P |  |  |  |  | P |
| $FP_{z'}$ | F |  |  | P | P |  |
| $C_z$ |  |  |  |  | P | P |
| $P_z$ | P | P |  | P |  |  |

In this matrix, P refers to monopolar power and F refers to mean frequency. The raw measures are averaged over 32 overlapped-epochs.

**ii) Components for the Discriminant Observer**

The specific set of raw components calculated in the PSA 4000 Algorithm are as follows:

Table 2:Raw-components used in calculating the probability of correct classification

| Raw-Component # | QUANTITY | CHANNEL/BAND(S) |
|---|---|---|
| 1 | monop abs power | $FP_1Tot$ |
| 2 | mean frequency | $FP_z.Tot$ |
| 3 | monop abs power | $P_z\alpha$ |
| 4 | power assymetry | $FP_1\ C_z\ \beta_2$ |
| 5 | monop power | $FP_z.\alpha$ |
| 6 | relative power | $P_z\Delta$ |
| 7 | monop power | $FP_z.\beta$ |
| 8 | monop power | $C_z\beta$ |
| 9 | monop abs power | $FP_1\beta_2$ |

The first 8 are used in calculating the PCC. The 9th is used in calculating the final EMG Index output parameter.

Z-components are obtained from the raw components by norming to a set of population means and standard deviations, which are obtained for each component from an experimental study of normative populations.

**iv) Z-Scores**

Z scores are either linear combinations of Z components, or identical to the z components. The z-score set used in the PSA 4000 discriminant is:

Table 3: Z-score set used in calculating the probability of correct classification

| Zscore # | Definition in terms of Z-components |
|----------|-------------------------------------|
| 1 | monop power ($FP_1Tot$) |
| 2 | mean frequency ($FP_z.Tot$) |
| 3 | monop power ($FP_z.\alpha$) - monop power ($P_z\alpha$) |
| 4 | power assymetry ($FP_1\ C_z\ \beta_2$) |
| 5 | relative power ($P_z\Delta$) |
| 6 | monop power ($FP_z.\beta$) - monop power ($C_z\beta$) |

The z-scores are linearly combined with weights such that the linear combination will maximize the separation between the two statistical groups: a group of aware people and a group of anesthetized unaware people.

**v) Calculation of the Probability of Correct Classification**

The probability that a set of z-scores can be correctly classified as belonging to an aware group, as opposed to an anesthetized unaware group, is obtained from the discriminant by using a sleep term,

$$S = c_s + \sum_{i=1}^{6} w_i^{(S)} z_i \qquad \textbf{17}$$

where $c_s$ is a constant term and the w's are discriminant weights. Similarly, the wake term is

$$W = c_w + \sum_{i=1}^{6} w_i^{(W)} z_i \qquad \textbf{18}$$

The probability of correct classification is

$$PCC = \frac{e^W}{e^W + e^S}$$

**19**

The PCC, calculated every 1.25 seconds, is a rigorously defined mathematical probability, and as such, varies between zero and one.

12) Observer Mediation

Observation mediation logic **25** mediates between the different observers and indices to produce a final set of output parameters, including the PSI.

**a) The Initial PSI**

The initial PSI is the starting point for observer mediation logic and is simply a linear range expansion of the PCC by a factor of 100.

**b) Variability Transformation of the initial PSI**

The initial PSI **152,** also referred to as the oPSI, undergoes a piecewise-linear transformation **153** that re-scales it according to the following formula:

$$rPSI = 1.7(oPSI) - 70.0 \qquad oPSI \geq 85.0 \qquad \textbf{20}$$

$$rPSI = 0.7(oPSI) + 15.0 \qquad 85.0 > oPSI > 15.0$$

$$rPSI = 1.7(oPSI) \qquad 15.0 > oPSI$$

The result is termed the rPSI.

**c) Mediation of Eye Blink Information**

Eye blink information is incorporated into the PSI **154** only prior to LOC, and only if the rPSI is greater than the LOC threshold of 50. An epoch is considered an eye blink epoch only if there are also no other types of artifact detected.
If an eye blink epoch is detected during an Indeterminate Probability (i.e., before an rPSI can be calculated, because the raw measure buffer is not yet full) then an rPSI of 95 is reported. This rPSI and all succeeding rPSI values (until the buffer is full enough to calculate a value) are then treated as if they originated from a calculated probability, i.e., it undergoes the transformations and calculations that lead to the PSI. After this first eye blink, whenever an eye blink is detected, the current rPSI is averaged with 99, and the result becomes the current rPSI.

**d) Mediation of Suppression Information**

The new PSI that includes the information represented by the pSR is referred to as the nPSI. The nPSI is constructed as a function of the rPSI and the pSR, denoted as nPSI(pSR,rPSI) **144.**
The transformation when pSR = 0, i.e., nPSI(0, rPSI), is an important special case of the whole transformation. Its result is a compression of the rPSI range (0-100) into a new scale. The new, compressed scale has a minimum value, $nPSI_{bot}$, so that range of the new scale is ($nPSI_{bot}$ - 100). The re-scaling is chosen so that a rPSI of 15 maps to a nPSI of 25. This completely determines the transformation equation, because the maxima of the two scales are the same. The transformation is given by

$$nPSI(0, rPSI) = \frac{15}{17}rPSI + nPSI_{bot} \qquad \textbf{21}$$

where $nPSI_{bot}$ is given by

$$nPSI_{bot} = 25 - \frac{15^2}{17} \cong 11.7647$$

Another condition is the special case at pSR= 15, i.e., nPSI(15, rPSI). We impose the condition

$$nPSI(pSR = 15, rPSI \geq 15) = 15 \qquad\qquad \textbf{23}$$

A third condition determining the transformation is case when $pSR \geq 50$. Then we have

$$nPSI(pSR \geq 50, rPSI) = 0 \qquad\qquad \textbf{24}$$

**e) Mediation of Beta5 Information**

Incorporation of EMG information into the PSI is based on the $F_1\beta_5$ index **140**. This modification is a refinement of the EMG information already in the PSI by virtue of the $F_1\beta_2$ term. EMG modifications are possible only when the pSR = 0, and only after a time threshold of 15 minutes after the declaration of Loss Of Consciousness (LOC). EMG modifications are not possible when the patient module is disconnected. In addition, there is a timeout period of 1/3 minute after the *end* of the PM Disconnect during which EMG modifications are not possible. When a BAD IMPEDANCE is detected, the EMG power used is the last one calculated before the BAD IMPEDANCE condition.

The PSI that incorporates possible changes due to the $F_1\beta_5$ index is called the tPSI. (The tPSI is a function of EMG and the nPSI.)

When the PSI is modified by the $F_1\beta_5$ index then the tPSI is considered a good data point and is painted non-white, even though the underlying PSI may have been artifacted and would have otherwise been painted white. The PSI is considered modified by EMG only when the change is greater than 1.

The change from nPSI to tPSI is calculated using the following equation: we express the change as the product of three functions:

$$\Delta PSI = f(F1\beta5)g(nPSI)h(F1\beta5, nPSI) \qquad\qquad \textbf{25}$$

The first function governs the rise along the $F_1\beta_5$ axis:
functions:

$$f(F1\beta5) = \left( \frac{1}{1 + e^{-(F1\beta5 - B_m)/B_w}} \right) \qquad\qquad \textbf{26}$$

The second function governs the rise along the nPSI axis:

$$g(nPSI) = \left( \frac{1}{1 + e^{-(nPSI - P_m)/P_w}} \right) \qquad\qquad \textbf{27}$$

The third function limits the change as nPSI becomes bigger, because there is a smaller remaining range of tPSI into which to change:

$$h(F1\beta5, nPSI) = t_c - \frac{t_c}{n_c + \dfrac{100 - n_c}{1 + e^{-(F1\beta5 - b_m)/b_w}}} nPSI \qquad\qquad \textbf{28}$$

In each of the three functions there is a functional form $F(x) = (1 + \exp((x+c)/d))^{-1}$. This function creates a rising transition from 0 to 1, with the midpoint of the transition occurring at x=c, and the width (or sharpness) of the transition determined by d.

Thus the first of the three equations above defines the contribution of the $FP_1\beta_5$ index as rising from zero around an index of approximately 1.25, and the second factor defines that the *change* can only occur be significant at nPSI values starting around a value of 19.

The third factor in the equation embodies the idea that as the nPSI gets larger, there is a smaller and smaller remaining range into which the tPSI can change. The change is limited to only part of this remaining range. The maximum of this limiting part is defined by the last term which also has the functional form of f(x). The maximum is itself a rising function of the $FP_1\beta_5$ index with a midpoint at a large value of 10.25 and a relatively large transition width of 3.0. This means that for most typical values of the $FP_1\beta_5$ index, the change is limited to a maximum value of about 80. This maximum will increase as the $FP_1\beta_5$ index increases.

The tPSI is given by

$$tPSI = nPSI + \Delta PSI \qquad\qquad \mathbf{29}$$

The EMG B5 baseline is adaptively determined as follows: The EMG B5 index over the past three minutes is stored in two windows, or buffers. The first holds the indices for the oldest two minutes (of the three minutes) and the second holds the most recent 1 minute (of the three minutes). For each of these windows, the averages and standard deviations are calculated at every update (every 1.25 seconds).

Every update, the following conditions on the averages and standard deviations are checked:

$$\langle F_1\beta_s \rangle_1 < A_1 \qquad\qquad \mathbf{30}$$

$$\left| \langle F_1\beta_s \rangle_1 - \langle F_1\beta_s \rangle_2 \right| < D \qquad\qquad \mathbf{31}$$

$$\sigma_2 < S_2 \qquad\qquad \mathbf{33}$$

$$\sigma_1 < S_1 \qquad\qquad \mathbf{32}$$

If all four conditions are satisfied, then the adaptive baseline, L, is set to the average in the first window, $A_1$:

$$L = A_1 \qquad\qquad \mathbf{34}$$

The variable A in the equation above has a value of A = 1.25 - baseline. The baseline in the beginning of the case is set to zero (the population baseline, since the EMG B5 z-scores used have been normalized to the population baseline). However the EMG B5 term is continuously monitored for conditions that will allow the setting of a new baseline. This allows the adaptation of EMG B5 modifications to individual patient differences. The adaptive baseline algorithm is described later in this section.

**f) Mediation of Artifact Information**

**i) Repeated PSI**

The conditions for declaring a Repeated PSI are distinct from the conditions for Repeated Probabilities. A Repeated Probability is generated if artifacts make an FFT unavailable. However, several things can cause the PSI to vary even if the underlying probability is a repeated probability. First, eye blink information can modify the oPSI'. Secondly, the nPSI can be calculated from repeated probabilities. If the pSR happens to change during repeated probabilities, the nPSI will vary even if the underlying probability does not. Third, EMG modifications can also be active during repeated probabilities.

The following conditions are used in the declaration of a Repeated PSI: if an epoch is an artifacted epoch, AND the PSI has NOT been modified by either eye blinks or EMG, AND the Artifact Index is greater than 30, then the tPSI is a Repeated PSI.

"Repeated PSI" is merely terminology, carried over from "Repeated Probability". It does not imply that the PSI is actually repeated, though in most cases it will be.

### ii) Artifacted PSI

Artifacted PSI's are distinct from Repeated PSI's. The Artifacted PSI declaration is made on the 2.5-second PSI values. Repeated 1.25-second PSI's or repeated full-epoch PSI's are possible (through the rules in the previous sections). A 2.5-second PSI is declared an Artifacted PSI if the 2.5 second PSI is a repeated PSI AND the Artifact Index is greater than 30.

### iii) Trend PSI

The Trend PSI is the running average of four 2.5 second PSI's, whether it is an Artifacted PSI or not. In the beginning of the case, the initial value of the running average is the population value of 95 for awake patients. The Trend PSI is the one of the four output parameters of the PSI 4000 Algorithm.

### *SYSTEM STATES AND SWITCHING*

As previously noted, the system operates in four distinct modes, each operating significantly differently. The four specifically defined states are as follows.

### *1) States*

### a) Data Accumulation

During Data Accumulation, the raw measure buffer of raw measure sets does has less than 24 overlapped-epochs of raw measure sets stored. EEG data acquisition is being performed, artifact analysis is being done, and FFT calculations are being made on good data. Each calculated raw measure set is added to the raw measure buffer. The following are NOT calculated: raw components, Z components, the PCC, and the PSI, and the EMG Index. The SR and the Artifact Index are calculated during this period.

### b) Awake State

In Awake State, the raw measure buffer has 24 or more (up to 32) overlapped-epochs of raw measure sets stored. EEG data acquisition is being performed, artifact analysis is being done, and FFT calculations are being made on good data. If the raw measure buffer has 32 raw measure sets, the oldest raw measure set is thrown away before the most recently calculated measure set is added. During this mode, all four output parameters are calculated from non-artifacted data. The most notable feature of this mode is the incorporation of eye blink information from artifact analysis into the PSI.

### c) Unconscious State

In Unconscious State, eye blink information is ignored and is NOT incorporated into the PSI. Otherwise, the operation of the Algorithm is the same as in Awake State.

### *1) Transition Events*

The following three transition events initiate a transition between the four states.

### a) Sufficient Data Accumulated

The Algorithm determines that sufficient data has been accumulated after the raw measure buffer has accumulated 24 overlapped-epochs of raw measure sets calculated from FFT data. FFT data can only be calculated from non-artifacted epochs. Thus the time spent in this state is variable, depending on the amount of artifacts present in the data.

**b) Loss of Consciousness**

If the PSI has 18 consecutive non-repeated values below 50, Loss Of Consciousness is declared. This declaration is used to disable the incorporation of eye blink information into the PSI.

**Claims**

**1.** An apparatus for classifying the level of awareness or anesthesia of a patient using electroencephalograph (EEG) signals, comprising:

> a. a plurality of patient electrodes whereby a plurality of patient EEG signals is acquired;
> b. a patient module connected to the patient electrode set; and
> c. an analysis unit connected to the patient module, said analysis unit comprising:
>
>> i. a plurality of modules comprising observers configured to produce measures of specific characteristics in the plural EEG signals, and
>> ii. an observer mediator which mediates among plural outputs of the observers according to a mediation logic, whereby the observer mediator produces at least one output parameter characterizing the patient's state of awareness or anesthesia; **characterized in that**

one of the modules comprising an observer comprises a subsystem which constructs and sends to the observer mediator a statistical discriminant based on plural statistical variables derived from power and frequency information extracted from plural EEG signals; and **in that** the plural modules comprising observers, other than the module comprising a subsystem which constructs a statistical discriminant, are plural classifiers which detect signatures within the plural EEG signals, which signatures are different from the statistical variables used to construct the statistical discriminant.

**2.** The apparatus of claim 1 in which the observer mediator weighs the output of the plural observers by monitoring each observer's input signal quality and the context of the observation based on the patient's state of awareness or anesthesia.

**3.** The apparatus of claim 1 in which one of the at least one output parameters is a Patient State Index which characterizes the patient's state of awareness or anesthesia.

**4.** The apparatus of claim 1 in which the plural classifiers comprise a corresponding plurality of artifact detectors.

**Patentansprüche**

**1.** Vorrichtung zum Klassifizieren des Wachheits- oder Narkosegrads eines Patienten mit Hilfe von Elektroenzephalogramm- (EEG) Signalen mit:

> a. mehreren Patientenelektroden, wodurch mehrere EEG-Signale des Patienten erfaßt werden;
> b. einem Patientenmodul, das mit dem Patientenelektrodensatz verbunden ist; und
> c. einer Analyseneinheit, die mit dem Patientenmodul verbunden ist, wobei die Analyseneinheit aufweist:
>
>> i. mehrere Module mit Beobachtern, die so konfiguriert sind, daß sie Messungen spezifischer Kennwerte in den mehreren EEG-Signalen erzeugen, und
>> ii. einen Beobachtervermittler, der zwischen mehreren Ausgaben der Beobachter gemäß einer Vermittlungslogik vermittelt, wodurch der Beobachtervermittler mindestens einen Ausgabeparameter erzeugt, der den Wachheits- oder Narkosezustand des Patienten **kennzeichnet;**

**dadurch gekennzeichnet, daß**

eines der Module einen Beobachter mit einem Teilsystem aufweist, das eine statistische Diskriminante auf der Grundlage mehrerer statistischer Variablen erstellt und zum Beobachtervermittler sendet, die aus Leistungs- und Frequenzinformationen abgeleitet sind, die aus mehreren EEG-Signalen extrahiert sind; und

**dadurch**, daß die mehreren Beobachter aufweisenden Module mit Ausnahme des Moduls mit einem Teilsystem, das eine statistische Diskriminante erstellt, mehrere Klassifizierer sind, die Signaturen in den mehreren EEG-Signalen detektieren, wobei sich die Signaturen von den statistischen Variablen unterscheiden, die zum Erstellen der statistischen Diskriminante verwendet werden.

2. Vorrichtung nach Anspruch 1, wobei der Beobachtervermittler die Ausgabe der mehreren Beobachter durch Überwachen der Eingangssignalqualität jedes Beobachters und des Kontextes der Beobachtung auf der Grundlage des Wachheits- oder Narkosezustands des Patienten gewichtet.

3. Vorrichtung nach Anspruch 1, wobei einer des mindestens einen Ausgabeparameters ein Patientenzustandsindex ist, der den Wachheits- oder Narkosezustand des Patienten kennzeichnet.

4. Vorrichtung nach Anspruch 1, wobei die mehreren Klassifizierer entsprechende mehrere Artefaktdetektoren aufweisen.

## Revendications

1. Appareil de classification du niveau de vigilance ou d'anesthésie d'un patient utilisant des signaux d'électroencéphalographe (EEG), comprenant :

> a. une pluralité d'électrodes « patient » au moyen desquelles une pluralité de signaux d'EEG du patient est acquise ;
> b. un module « patient » relié à l'ensemble d'électrodes « patient » ; et
> c. une unité d'analyse reliée au module « patient », ladite unité d'analyse comprenant :
>
> > i. une pluralité de modules comprenant des observateurs configurés pour produire des mesures de caractéristiques spécifiques dans les multiples signaux d'EEG, et
> > ii. un médiateur d'observateurs qui sert de médiateur entre les multiples sorties des observateurs selon une logique de médiation, moyennant quoi le médiateur d'observateurs produit au moins un paramètre de sortie caractérisant l'état de vigilance ou d'anesthésie du patient ; **caractérisé en ce que**

l'un des modules comprenant un observateur comprend un sous-système qui construit et transmet au médiateur d'observateurs une discriminante statistique basée sur de multiples variables statistiques dérivées d'informations de puissance et de fréquence extraites à partir des multiples signaux d'EEG ; et

**en ce que** les multiples modules comprenant des observateurs, autres que le module comprenant un sous-système qui construit une discriminante statistique, sont de multiples classificateurs qui détectent des signatures au sein des multiples signaux d'EEG, lesquelles signatures sont différentes des variables statistiques utilisées pour construire la discriminante statistique.

2. Appareil selon la revendication 1, dans lequel le médiateur d'observateurs pondère la sortie des multiples observateurs en surveillant la qualité du signal d'entrée de chaque observateur et le contexte de l'observation sur la base de l'état de vigilance ou d'anesthésie du patient.

3. Appareil selon la revendication 1, dans lequel l'un desdits au moins un paramètre de sortie est un indice d'état du patient qui caractérise l'état de vigilance ou d'anesthésie du patient.

4. Appareil selon la revendication 1, dans lequel les multiples classificateurs comprennent une pluralité correspondante de détecteurs d'artefact.

## Multiple Observer Derived Measurement Model

**Physiological System** — 1

**Signal Acquisition and Filtering System** — 2

**Observer A** — 3

**Observer D**

**Observer B**

**Observer C**

**Observer Mediator** — 4

Patient State

**Patient State Logic** — 5

**Derived Parameter** — 6

*Figure 1*

Figure 2

## PSA 4000 Configuration

**Patient Electrode Set**

43

Patient Cable

44

42

Analog Board

**Patient Module**

Digital Board

Patient Module Cable

40

41

| Power | Computer Subsystem | | Export Data Archive |
|---|---|---|---|
| | User Input | Display | |
| **Host Instrument** | | | |

Figure 3

Figure 4

Figure 5

**1.25 Processed Parameter Packet @ every 1.25 second** — 137

158 — Artifact Index

155 — Artifact Type

145 — FFT Data Available — **Yes** — Complex Results — 146 — **No**

141 — SRcode

Beta5 Power — 126

149 — Discriminant Processing

Beta2z Processing

138 — Sampling Algorithm

156 — **Yes** — Before LOC — **No**

150 — **No** — Update Discriminant — **Yes**

147 — 142 — Compute 2.5 Minute Suppression Ratio

139 — Adaptive Baseline

157 — Only Eye Blinks — **No** — **Yes**

151 — Compute New LastValidPsi

1-minute EMG-Index — 148

Compute One-Minute Suppression Ratio — 143

152 — LastValid Psi (opsi)

153 — T6/Range Mapping — OR — oPsi,SR Mapping — 144

154 — Eye Blink Modification — OR

OR

OR — 140 — Beta5z/nPsi Mapping

**2.5 Processed Parameter Message Packet @ every 2.5 seconds** — 160

Psi Trend Averaging — 161

1 Minute Artifact_Index — 165

Psi Trend Value — 164

1 Minute EMG_Index — 163

1 Minute SR_Index — 162

Figure 6

Figure 7:

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5813993 A
- US 5816247 A
- US 5775330 A
- US 5699808 A
- US 4557270 A
- US 4545388 A
- US 5083571 A
- US 5377100 A
- US 6067467 A
- US 09113946 B
- US 5479934 A